(19) Europäisches Patentamt
European Patent Office
Office européen des brevets

(11) Veröffentlichungsnummer: **0 308 794 B1**

(12) **EUROPÄISCHE PATENTSCHRIFT**

(45) Veröffentlichungstag der Patentschrift: **06.04.94**

(51) Int. Cl.[5]: **C07D 239/26**, C07D 239/34, C09K 19/34

(21) Anmeldenummer: **88115002.3**

(22) Anmeldetag: **14.09.88**

(54) **Flüssigkristalline Cyclohexancarbonsäurephenylpyrimidinester mit smektischer Phase in Flüssigkristall-Mischungen und ihre Verwendung.**

(30) Priorität: **19.09.87 DE 3731639**

(43) Veröffentlichungstag der Anmeldung:
**29.03.89 Patentblatt 89/13**

(45) Bekanntmachung des Hinweises auf die Patenterteilung:
**06.04.94 Patentblatt 94/14**

(84) Benannte Vertragsstaaten:
**AT BE CH LI SE**

(56) Entgegenhaltungen:
**EP-A- 0 025 119**
**EP-A- 0 056 501**
**EP-A- 0 151 446**
**EP-A- 0 181 601**
**EP-A- 0 268 198**
**EP-A- 0 293 910**
**WO-A-86/07085**
**WO-A-87/01701**
**GB-A- 2 092 169**
**JP-A-60 149 564**

(73) Patentinhaber: **HOECHST AKTIENGESELLSCHAFT**

**D-65926 Frankfurt(DE)**

(72) Erfinder: **Wingen, Rainer, Dr.**
**Rotkäppchenweg 10**
**D-6234 Hattersheim am Main(DE)**
Erfinder: **Dübal, Hans-Rolf, Dr.**
**Heuhohlweg 6**
**D-6240 Königstein/Taunus(DE)**
Erfinder: **Escher, Claus, Dr.**
**Amselweg 3**
**D-6109 Mühltal(DE)**
Erfinder: **Hemmerling, Wolfgang, Dr.**
**Billtalstrasse 32**
**D-6231 Sulzbach (Taunus)(DE)**
Erfinder: **Müller, Ingrid, Dr.**
**Am Pfingstbrunnen 1**
**D-6238 Hofheim am Taunus(DE)**
Erfinder: **Ohlendorf, Dieter, Dr.**
**Am Kühlen Grund 4**
**D-6237 Liederbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 308 794 B1

**Beschreibung**

Ferroelektrische Flüssigkristalle haben in jüngerer Zeit wegen ihrer günstigen Eigenschaften - wie kurze Schaltzeiten, Möglichkeit des bistabilen Schaltens und nahezu blickwinkelunabhängiger Kontrast - Interesse als Anzeigemedium für elektro-optische Bauteile gewonnen.

Ferroelektrische Flüssigkristalle können selbst chirale Verbindungen sein, die chirale smektische, insbesondere $S_c^*$ -phasen ausbilden. Man erhält aber auch ferroelektrische Flüssigkristall-Mischungen, indem man Verbindungen oder Mischungen, die selbst nicht chiral aufgebaut sind, aber geneigt-smektische Phasen ausbilden, mit chiralen Verbindungen dotiert [M. Brunet, Cl. Williams, Ann. Phys. 3, 237 (1978)].

Für den praktischen Einsatz solcher Flüssigkristall-Mischungen ist es zunächst erforderlich, daß die smektische Phase, insbesondere die $S_c^*$ -Phase über einen breiten Temperaturbereich stabil ist. Um ein hohes Kontrastverhältnis in elektro-optischen Bauelementen zu erzielen, ist ferner eine einheitliche planare Orientierung der Flüssigkristalle nötig. Es ist bekannt, daß sich eine einheitliche planare Orientierung in der $S_c^*$ -Phase erreichen läßt, wenn die Phasenfolge des Flüssigkristallsystems mit abnehmender Temperatur lautet

Isotrop$\rightarrow N^* \rightarrow S_A^* \rightarrow S_c^*$.

(z.B. T. Matsumoto et al., Proc. of the 6th Int. Display Research Conf., Japan Display, 30. Sept. - 2. Okt. 1986, Tokyo, pages 468 - 470; M. Murakami et al., loc. cit. pages 344 - 347). Häufig bilden nun flüssigkristalline Verbindungen oder Systeme mit breiter $S_c$-Phase keine oder nur eine sehr enge nematische Phase. Der Zusatz einer Verbindung mit breiter nematischer Phase zu solchen $S_c$-Verbindungen oder -Systemen beeinträchtigt die $S_c$-Phase oder bringt sie zum Verschwinden.

Es wurde nun gefunden, daß trans-Cyclohexancarbonsäurephenylpyrimidinester der allgemeinen Formel (I)

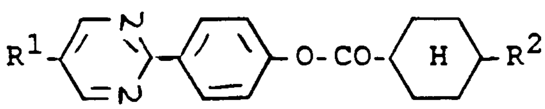

(I),

in der $R^1$ einen Alkylrest mit 10 bis 16 C-Atomen, insbesondere 11 bis 16 C-Atomen, oder ein Alkyloxyrest mit 8 bis 14 C-Atomen und $R^2$ einen Alkylrest mit 2 bis 9 C-Atomen bedeuten, neben für praktische Zwecke ausreichend breiten nematischen Phasen auch smektische, insbesondere $S_c$-, Phasen in teilweise bemerkenswert breiten Temperaturbereichen ausbilden.

Aus der EP-A 00 25 119 sind 4-Alkyl-cyclohexancarbonsäure-[4-(5-alkylpyrimidin-2-yl)]-phenylester bekannt, bei denen in den konkreten Beispielen die Alkylsubstituenten fünf bzw. sechs C-Atome enthalten. Ein Hinweis auf derartige Verbindungen findet sich ferner in EP-A 01 51 446 und in WO-A 86/07055. Soweit in diesen Veröffentlichungen überhaupt konkrete Verbindungen angegeben werden, weisen diese jedoch keine smektische Phase auf. Das Auftreten einer smektischen C-Phase neben einer relativ breiten nematischen Phase bei den erfindungsgemäßen Verbindungen war daher nicht zu erwarten. Es war vielmehr anzunehmen, daß in dieser Verbindungsklasse nur eine nematische Phase auftritt.

In WO 86/07085 sind mit Stickstoff haltigen Heterocyclen substituierte Cyclohexancarbonsäure-phenylester als Komponenten smektischer Flüssigkristall mischungen beschrieben. Konkrete Verbindungen werden jedoch nicht angegeben und es gibt keinen Hinweis daß bestimmte Verbindungen aus dieser Gruppe nematische Phasen in smektischen Flüssigkristall mischungen induzieren oder Verbreitern können.

Gegenstand der Erfindung ist eine flüssigkristalline Mischung, dadurch gekennzeichnet, daß sie mindestens einen trans-Cyclohexancarbonsäure-phenylpyrimidinester der allgemeinen Formel (I) enthält,

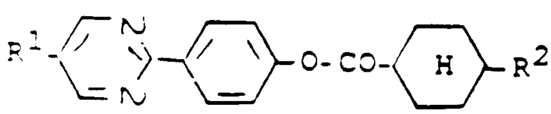

(I),

in der $R^1$ einen Alkylrest mit 10 bis 16 C-Atomen, insbesondere 11 bis 16 C-Atomen ein Alkyloxyrest mit 8 bis 14 C-Atomen und $R^2$ einen Alkylrest mit 2 bis 9 C-Atomen bedeuten, und neben einer smektischen C / ferroelektrischen Phase auch eine nematische Phase ausbildet.

Die Verbindungen der Formel I) sind thermisch, chemisch und photochemisch stabil. Man erhält sie aus den zugrundeliegenden 4-(5-Alkyl- bzw. 5-Alkoxy-pyrimidin-2-yl)-phenolen oder ihren Alkali- oder Erdalkalisalzen durch Umsetzung mit entsprechenden 4-Alkyl-(trans)-cyclohexancarbonsäuren oder deren Halogeniden, insbesondere Chloriden.

Vorzugsweise werden zur Herstellung der Verbindungen (I) die Phenole und die Säurechloride eingesetzt, wobei die Umsetzung in Gegenwart von Säurefängern wie Aminen, beispielweise Pyridin oder Triethylamin, oder von Erdalkali- oder Alkali(hydrogen)-carbonaten im allgemeinen bei Temperaturen zwischen -40 und +70 °C erfolgt. Es ist aber auch möglich, die Phenole mit den Säuren umzusetzen, und zwar in Gegenwart von Brönstedt- oder Lewissäuren, ggf. in Gegenwart wasserbindender Mittel oder unter physikalischer Entfernung des Reaktionswassers, z.B. durch azeotrope Destillation oder Absorption, oder unter Zuhilfenahme von Kondensationsreagenzien wie N-N'-Carbonyldiimidazol, Dicyclohexylcarbodiimid oder Azodicarbonsäureester/Triphenylphosphin. Es ist auch möglich, die Alkali- oder Erdalkalisalze der Phenole mit den Carbonsäurehalogeniden, insbesondere den Chloriden, umzusetzen. Das jeweils erhaltene Rohprodukt kann in an sich bekannter Weise gereinigt werden, beispielweise durch Umkristallisieren oder durch Säulenchromatographie.

Die Verbindungen der Formel (I) eignen sich besonders gut als Komponenten smektischer flüssigkristalliner Mischungen, da sie sowohl nematische als auch smektische C-Phasen aufweisen. Gibt man die Verbindungen zu Mischungen aus Flüssigkristallen oder auch zu einzelnen flüssigkristallinen Verbindungen, die eine $S_c$- und $S_A$-Phase, aber keine oder nur eine sehr schmale nematische Phase besitzen, so induzieren oder vergrößern die neuen Verbindungen schon bei Zugabe geringer Mengen, ab etwa 4 Mol-% in der Mischung, die gewünschte nematische Phase und vergrößern gleichzeitig den Temperaturbereich der $S_c$-Phase. Besonders bemerkenswert ist, daß die Verbreiterung der $S_c$-Phase häufig nicht nur zu tiefen Temperaturen erfolgt, sondern auch eine Verschiebung der $S_c/S_A$-Phasengrenze zu höheren Temperaturen auftritt (Beispiele 10 bis 13). Natürlich ist es auch möglich, die erfindungsgemäßen Verbindungen miteinander zu mischen, um so eine Verbreiterung der $S_c$-Phase, insbesondere eine Absenkung der unteren Temperaturgrenze im Vergleich zu den Ausgangsverbindungen, zu erzielen. Man kann daher auch optimierte Mischungen aus den Verbindungen der Formel (I) als Komponenten ferroelektrischer/smektisch C-Mischungen verwenden.

Gegenstand der Erfindung ist daher auch die Verwendung der Verbindungen, der Formel (I) einzeln oder als Mischungen untereinander, als Komponenten flüssigkristalliner smektisch C bzw. flüssigkristalliner ferroelektrischer Mischungen zwecks Induzierung oder Verbreiterung einer flüssigkristallinen nematischen Phase, wobei der Temperaturbereich der smektischen C-Phase meistens nicht verkleinert, sondern häufig zu tiefen und insbesondere zu hohen Temperaturen vergrößert wird.

Weiterhin vorteilhaft für die Verwendung der Verbindungen der Formel (I) in ferroelektrischen flüssigkristallinen Mischungen ist, daß sie eine negative dielektrische Anisotropie (vgl. Beispiel 14) und eine sehr kleine optische Anisotropie (vgl. Beispiel 15) aufweisen. Hierdurch werden die dielektrischen und optischen Eigenschaften von Mischungen günstig beeinflußt (vgl. Lagerwall et al., "Ferroelectric Liquid Crystals for Displays", SID Symposium, Oct. Meeting 1985, San Diego, Ca. USA).

Weiterhin Gegenstand der Erfindung ist ein elektro optisches Bauteil, enthaltend eine flüssigkristalline Mischung, die mindestens eine Verbindung der allgemeinen Formel (I) enthält.

**Beispiel 1**

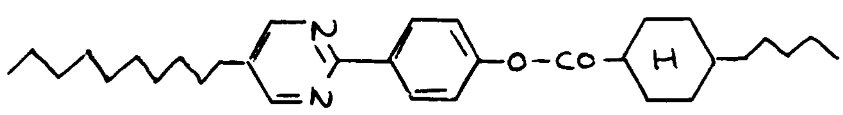

trans-4-Pentyl-cyclohexancarbonsäure-[4-(5-decyl-pyrimidin-2-yl)]phenyl-ester

Eine Lösung von 35,4 g 4-(5-decyl-pyrimidin-2-yl)phenol 22,5 g trans-4-Pentyl-cyclohexancarbonsäure und 23,4 g N,N'-Dicyclohexylcarbodiimid in 600 ml Dichlormethan wird 12 h bei 20 °C gerührt. Der gebildete N,N'-Dicyclohexylharnstoff wird abfiltriert und das Filtrat über 5 kg Kieselgel mit Dichlormethan chromatographiert. Die produkthaltige Fraktion ergibt nach Umkristallisation aus n-Hexan 21,5 g farblose Kristalle.

Phasenfolge K(10 $S_x$ 51,5) 62,8 $S_3$ 85,7 $S_c$ 104,5 N 162,4 I

Analog werden erhalten:

**Beispiel 2**

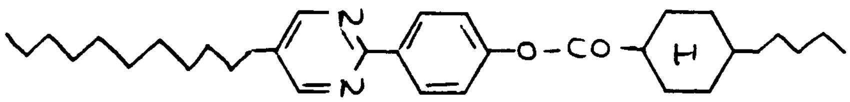

trans-4-Pentyl-cyclohexancarbonsäure-[4-(5-undecyl-pyrimidin-2-yl)]phenyl-ester

Phasenfolge K 70 $S_x$ 80 $S_2$ 82,7 $S_c$ 114,5 N 160,5 I

**Beispiel 3**

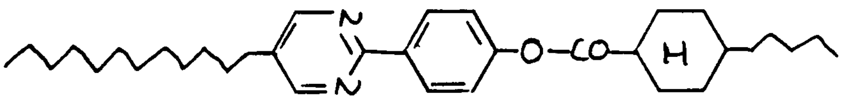

trans-4-Pentyl-cyclohexancarbonsäure-[4-(5-dodecyl-pyrimidin-2-yl)]phenyl-ester

Phasenfolge K 67 $S_3$ 83 $S_2$ 87,2 $S_c$ 121 N 156 I

**Beispiel 4**

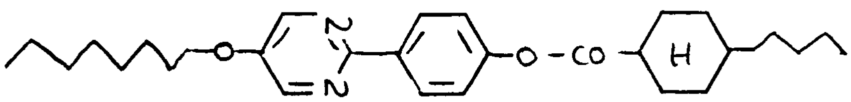

trans-4-Pentyl-cyclohexancarbonsäure-[4-(5-octyloxy-pyrimidin-2-yl)]phenyl-ester

Phasenfolge K (35 $S_x$ 68) 72,1 $S_3$ 74 $S_c$ 100 N 193 I

**Beispiel 5**

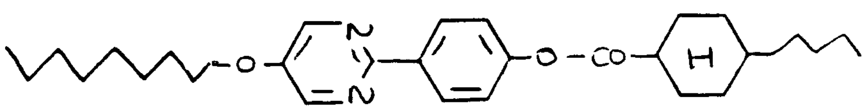

trans-4-Pentyl-cyclohexancarbonsäure-[4-(5-nonyloxy-pyrimidin-2-yl)]phenyl-ester

Phasenfolge K ($K_2$ 64 $S_G$ 69) 74,3 $S_c$ 117,7 N 189 I

**Beispiel 6**

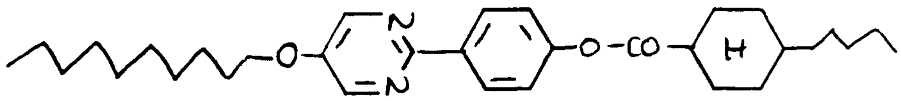

trans-4-Pentyl-cyclohexancarbonsäure-[4-(5-decyloxy-pyrimidin-2-yl)]phenyl-ester

Phasenfolge K ($K_2$ 65 $S_G$ 72,5) 74,7 $S_c$ 129,8 N 186,5 I

**Beispiel 7**

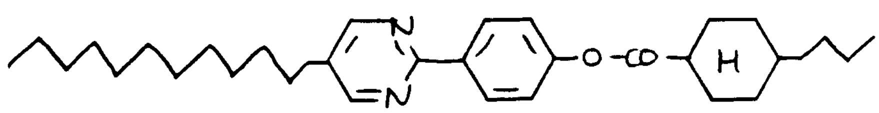

trans-4-Butyl-cyclohexancarbonsäure-[4-(5-dodecyl-pyrimidin-2-yl)]phenyl-ester

Phasenfolge K 80 $S_3$ 82 $S_2$ 83,8 $S_c$ 115,3 N 152,7 I

**Beispiel 8**

Eine binäre Mischung aus jeweils 50 Mol-% der beiden Verbindungen
trans-4-Pentyl-cyclohexancarbonsäure-[4-(5-octyloxy-pyrimidin-2-yl)]phenyl-ester (Beispiel 4)
trans-4-Pentyl-cyclohexancarbonsäure-[4-(5-undecyl-pyrimidin-2-yl)phenyl-ester (Beispiel 5)
zeigt eine Phasenfolge von
K 45 $S_2$ 70,7 $S_c$ 107,5 N 177,5 I.

**Anwendungsbeispiele 9 - 12**

Es werden binäre Mischungen hergestellt, von denen jeweils eine Komponente eine erfindungsgemäße Verbindung ist und die andere Komponente eine Verbindung mit $S_c$-Phase, die keine bzw. nur eine sehr schmale nematische Phase aufweist. Die verwendeten Komponenten mit $S_c$-Phase sind

I: 5-Decyl-2-(4-octyloxy-phenyl)-pyrimidin II: 5-Octyloxy-2-(4-octyloxy-phenyl)-pyrimidin

III: 5-Nonyloxy-2-(4-heptyloxy-phenyl)-pyrimidin IV: 4-(4-Decyloxy-phenyl-1-carbonyloxy)-1-(4-methylhexyloxy)-benzol

Tabelle I faßt die Ergebnisse zusammen. Angegeben sind jeweils die Phasenfolgen für die reinen Verbindungen I bis IV, sowie für die binären Mischungen.

Die Ergebnisse in Tablle I demonstrieren eindeutig die guten Mischungseigenschaften der neuen Verbindungen. In allen Beispielen wird, ausgehend von den Verbindungen I bis IV (Verbindung A), durch Zugabe von nur 15 Mol-% der erfindungsgemäßen Verbindungen (Verbindung B) in der Mischung A + B der Temperaturbereich der $S_c$-Phase vergrößert und eine nematische Phase induziert bzw. vergrößert.

**Tabelle I**

| Beispiel | Verbindung A: Phasenfolge*) | Verbindung B (Verb. des Beispiels..) | molares Mischungs-verhältnis A/B | Phasenfolge *) |
|---|---|---|---|---|
| 9 | I: K 40,1 $S_C$ 69,1 $S_A$ 74,7 I | Beispiel 6 | 85/15 | K 35 $S_C$ 78,6 $S_A$ 80,8 N 92 I |
| 10 | II: K 50,7 $S_C$ 92,3 $S_A$ 99,5 N 100,3 I | Beispiel 6 | 85/15 | K 44 $S_C$ 96,5 $S_A$ 100 N 109,2 I |
| 11 | III: K 56 $S_C$ 94,4 $S_A$ 97,5 I | Beispiel 4 | 85/15 | K 47 $S_C$ 98,6 $S_A$ 99,8 N 107 I |
| 12 | IV: K 45 $S_C$ 70,4 $S_A$ 73,3 I [17 $S_G$ 33 $S_C$] | Beispiel 1 | 85/15 | K 27,3 $S_C$ 72,4 $S_A$ 74,6 N 81 I |

*) Es bedeuten: K - kristallin, $S_C$ - smektisch C, $S_A$ - smektisch A,
N - nematisch, I - isotrop
die Zahlenwerte zwischen den Phasenbezeichnungen geben jeweils die Umwandlungstemperaturen in °C an.

**Beispiel 13**

Eine Mischung aus den Komponenten

| | | |
|---|---|---|
| 1) | 5-Octyloxy-2-(4-octyloxy-phenyl)-pyrimidin | |
| 2) | 5-Octyloxy-2-(4-hexyloxy-phenyl)-pyrimidin | 59,5 Mol-% |
| 3) | 5-Octyloxy-2-(4-butyloxy-phenyl)-pyrimidin | |
| 4) | 4-(4-Decyloxy-phenyl-1-carbonyloxy)-1-(4-methyl-hexyl-oxy)-benzol | 30 Mol-% |
| 5) | trans-4-Pentyl-cyclohexancarbonsäure-[4-(5-decyl-pyrimidin-2-yl)]phenyl-ester | 10,5 Mol-% |

zeigt folgendes Phasenverhalten:

K-1 $S_c$ 74,5 $S_A$ 83,8 N 92,5 I

Auch in diesem Beispiel wird durch Zugabe einer erfindungsgemäßen Verbindung (-Komponente 5) die Breite der nematischen Phase von 0,8 °C auf 8,7 °C vergrößert.

**Beispiel 14**

Von der Verbindung aus Beispiel 1 werden die Dielektrizitätskonstanten bestimmt. Diese betragen bei 95 °C und einer Meßfrequenz von 20 kHz: $\epsilon_\perp$ = -0,37 und $\epsilon_\parallel$ = -1,23, so daß sich für die dielektrische Anisotropie $\Delta\epsilon$ ein Wert von -0,86 ergibt.

**Beispiel 15**

Von den beiden Verbindungen aus Beispiel 1 und Beispiel 4 werden jeweils Mischungen mit 50 Mol-% der Verbindung V 4-(5-Octyl-pyrimidin-2-yl)-1-(6-methyl-octyloxy)benzol hergestellt und sowohl von den beiden Mischungen als auch von der Einzelverbindung V die optische Anisotropie, Δn, bei 50 °C bestimmt:

| | |
|---|---|
| Verbindung V: | Δn = 0,134 |
| Mischung mit Beispiel 1: | Δn = 0,108 |
| Mischung mit Beispiel 4: | Δn = 0,0974 |

Linear extrapoliert auf die reinen Verbindungen ergibt sich für die Verbindung aus Beispiel 1, Δn = 0,082, und aus Beispiel 4, Δn = 0,0608.

**Beispiel 16**

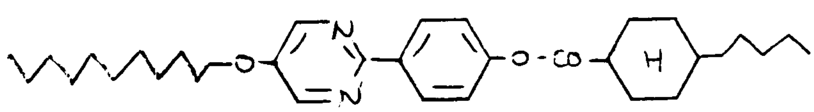

trans-4-Pentyl-cyclohexancarbonsäure-[4-(5-undecyloxypyrimidin-2-yl)]phenyl-ester

Phasenfolge K 80 $S_2$ 81 $S_c$ 136 N 181 I

**Beispiel 17**

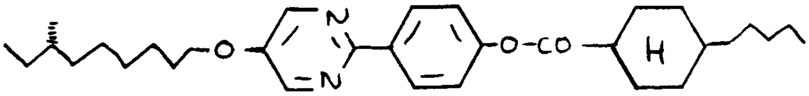

trans-4-Pentyl-cyclohexancarbonsäure-[4-<5-{(S)-7-methyl-nonyloxy}pyrimidin-2-yl>]phenyl-ester

Phasenfolge K 60 $S_2$ 84 $S_c$ 114 N 171 I
$[\alpha]_D^{25}$ : -3,75(c = 2,$CHCl_3$)

**Beispiel 18**

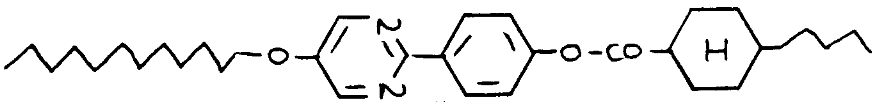

trans-4-Pentyl-cyclohexancarbonsäure-[4-(5-dodecyloxy-pyrimidin-2-yl)]phenyl-ester

Phasenfolge K 81 $S_2$ 90 $S_c$ 143 N 181 I

**Beispiel 19**

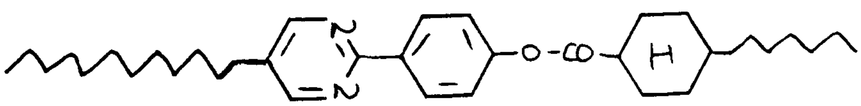

trans-4-Heptyl-cyclohexancarbonsäure-[4-(5-dodecylpyrimidin-2-yl)]phenyl-ester

Phasenfolge K 79,1 $S_2$ 87,2 $S_c$ 115 N 155,7 I

**Patentansprüche**

1. Flüssigkristalline Mischung, dadurch gekennzeichnet, daß sie mindestens einen trans-Cyclohexancarbonsäure-phenylpyrimidinester der allgemeinen Formel (I) enthält,

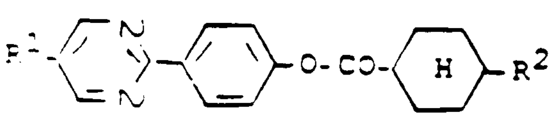 (I),

in der $R^1$ einen Alkylrest mit 10 bis 16 C-Atomen, oder ein Alkyloxyrest mit 8 bis 14 C-Atomen und $R^2$ einen Alkylrest mit 2 bis 9 C-Atomen bedeuten, und neben einer smektischen C/ferroelektrischen Phase auch eine nematische Phase ausbildet.

2. Flüssigkristalline Mischung nach Anspruch 1, dadurch gekennzeichnet, daß der R est $R^1$ in der allgemeinen Formel I einen Alkylrest mit 11 bis 16 C-Atomen bedeutet.

3. Elektrooptisches Bauteil, enthaltend eine flüssigkristalline Mischung nach Anspruch 1 oder 2.

4. Verwendung von trans Cyclohexancarbonsäure-phenylpyrimidinestern der allgemeinen Formel (I) in Anspruch 1 oder 2 einzeln oder als Mischungen untereinander, als Komponenten flüssigkristalliner smektisch C bzw. flüssigkristalliner ferroelektrischer Mischungen zwecks Induzierung oder Verbreiterung einer flüssigkristallinen nematischen Phase.

## Claims

1. A liquid-crystalline mixture which contains at least one phenylpyrimidinyl trans-cyclohexanecarboxylate of the formula (I)

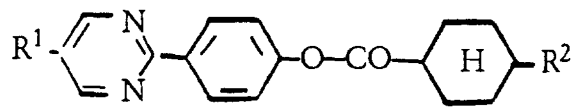

(I),

in which $R^1$ is an alkyl radical having 10 to 16 carbon atoms or an alkoxy radical having 8 to 14 carbon atoms, and $R^2$ is an alkyl radical having 2 to 9 carbon atoms, and, in addition to a smectic C/ferroelectric phase, also forms a nematic phase.

2. A liquid-crystalline mixture as claimed in claim 1, wherein the radical $R^1$ in the formula I is an alkyl radical having 11 to 16 carbon atoms.

3. An electro-optical component containing a liquid-crystalline mixture as claimed in claim 1 or 2.

4. The use of phenylpyrimidinyl trans-cyclohexane-carboxylates of the formula (I) in claim 1 or 2, individually or as mixtures with one another, as components of liquid-crystalline smectic C or liquid-crystalline ferroelectric mixtures for inducing or broadening a liquid-crystalline nematic phase.

## Revendications

1. Mélange de cristaux liquides, caractérisé en ce qu'il contient au moins un trans-cyclohexanecarboxylate de phénylpyrimidine de formule générale (I)

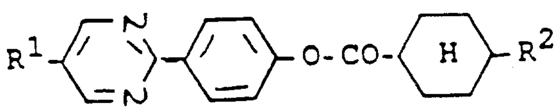

(I),

dans laquelle $R^1$ représente un reste alkyle de 10 à 16 atomes de carbone ou un reste alkyloxy de 8 à 14 atomes de carbone, et $R^2$ représente un reste alkyle de 2 à 9 atomes de carbone, et en ce qu'il forme aussi, en plus d'une phase smectique C/ferro-électrique, une phase nématique.

2. Mélange de cristaux liquides selon la revendication 1, caractérisé en ce que le reste $R^1$ de la formule générale (I) représente un reste alkyle de 11 à 16 atomes de carbone.

3. Composant électro-optique contenant un mélange de cristaux liquides selon la revendication 1 ou 2.

4. Utilisation de trans-cyclohexanecarboxylates de phénylpyrimidine de formule générale (I) de la revendication 1 ou 2, séparément ou sous forme de mélanges entre eux, comme constituants de mélanges de cristaux liquides smectiques C ou de cristaux liquides ferro-électriques pour l'induction ou l'élargissement d'une phase nématique de cristaux liquides.